Europäisches Patentamt

European Patent Office

Office européen des brevets

(11) Publication number: **0 177 832**
**B1**

## EUROPEAN PATENT SPECIFICATION

(45) Date of publication of patent specification: **26.04.89**

(21) Application number: **85112080.8**

(22) Date of filing: **24.09.85**

(51) Int. Cl.⁴: **B 01 J 23/85, C 07 C 15/46,
C 07 C 5/333**

(54) Dehydrogenation catalysts.

(30) Priority: **11.10.84 JP 213224/84**

(43) Date of publication of application:
**16.04.86 Bulletin 86/16**

(45) Publication of the grant of the patent:
**26.04.89 Bulletin 89/17**

(84) Designated Contracting States:
**DE FR GB IT**

(56) References cited:
**DE-B-1 643 916
FR-A-2 411 169
GB-A-1 464 252
US-A-4 467 046
US-A-4 496 662**

(73) Proprietor: **NISSAN GIRDLER CATALYST CO.,
LTD.
9-2, Kandata-cho 2-chome
Chiyoda-ku Tokyo (JP)**

(72) Inventor: **Hirano, Takenori
2-16, Yutaka-cho 5-chome
Kasukabe-shi Saitama-ken (JP)**
Inventor: **Unei, Hidemi
5-3, Akitsu-cho 1-chome
Higashimurayama-shi Tokyo (JP)**

(74) Representative: **Reitzner, Bruno, Dr.
Patentanwälte Dipl.-Ing. R. Splanemann Dr. B.
Reitzner, Dipl.-Ing. K. Baronetzky Tal 13
D-8000 München 2 (DE)**

Courier Press, Leamington Spa, England.

EP 0 177 832 B1

# EP 0 177 832 B1

**Description**

The present invention relates to dehydrogenation catalysts. More particularly, the present invention relates to magnesium oxide-containing dehydrogenation catalysts which are useful for a process for producing styrene by the dehydrogenation of ethylbenzene in the presence of steam and whereby styrene can be produced in good yield even when the molar ratio of steam to ethylbenzene (hereinafter referred to simply as the "steam to oil ratio") is low.

At present, styrene is produced on an industrial scale by the dehydrogenation of ethylbenzene in the presence of steam. The conventional dehydrogenation reaction by means of an industrial catalyst has been conducted in a substantial amount of steam with a steam to oil ratio of more than 12. The reasons for feeding steam are: (1) to lower the partial pressure of styrene so that the reaction proceeds advantageously under an equilibrium condition; (2) to remove carbonaceous matter which deposites on the catalyst surface during the dehydrogenation reaction, by a water gas reaction; and (3) to use the steam as a heat supply source.

In recent years, efforts have been made to reduce the consumption of steam, i.e. to decrease the steam to oil ratio, from the standpoint of saving energy.

Lowering the steam to oil ratio has an advantage that the cost of styrene can be reduced. However, on the negative side, it promotes the formation of carbonaceous matter at a dehydrogenation temperature of from 500 to 700°C. Thus, the carbonaceous matter tends to deposit on the catalyst, and there has been a difficulty that the conventional industrial catalyst undergoes deterioration in the catalytic activity to a substantial extent when the steam to oil ratio is 12 or less.

The conventional industrial catalyst is usually composed of iron oxide, a promoter (such as chromium or cerium) and an alkali metal or alkaline earth metal compound. The alkali metal or alkaline earth metal compound is incorporated for the purpose of promoting the water gas reaction and removing the carbonaceous matter deposited on the catalyst. Generally speaking, potassium, rubidium and cesium are effective. But potassium is most commonly used for the economical reason. The content of the alkali metal or alkaline earth metal compound is disclosed, for example, in Japanese Examined Patent Publicaton No. 19864/1968, Japanese Unexamined Patent Publication No. 7889/1977, No. 94295/1978, No. 129190/1978 or No. 129191/1978. When the alkali metal compound is used alone, the amount is normally at most 30 wt.%. Japanese Unexamined Patent Publication No. 25134/1972 discloses catalysts containing potassium as the alkali metal and calcium as the alkaline earth metal, wherein the total amount of the potassium and calcium compounds is at most 24 wt.%. Further, dehydrogenation catalysts such as Phillips 1490 and Standard 1707 that had been used in the past, contained from 30 to 45 wt.% and 72.4 wt.% of magnesium oxide, respectively, as a carrier for iron oxide which is the main active component.

As mentioned above, in the conventional industrial catalysts, the content of the alkali metal or alkaline earth metal compound added as a promoter for the water gas reaction, is at most 30 wt.%, whereby when the steam to oil ratio is 12 or less, the amount of the deposition of carbonaceous material increases, and the catalytic activity tends to decrease and deteriorate. As a method for preventing the deterioration of the catalytic activity at a low steam to oil ratio, there have been known with respect to the iron-chromium-potassium catalyst as a typical dehydrogenation catalyst, a method wherein the potassium content is increased to promote the water gas reaction and to readily remove the carbonaceous matter deposited on the catalyst, and a method werein the iron oxide is calcined at a high temperature of at least 800°C to prevent the formation of carbonaceous matter itself.

However, when left in air, catalysts containing a substantial amount of potassium tend to absorb water due to the highly strong hygroscopic nature of potassium, whereby the strength of the molded catalyst pellets is likely to deteriorate, potassium tends to gradually migrate towards the inner part of the pellets during the dehydrogenation reaction depending upon the reaction conditions, or potassium is likely to migrate out of the catalyst pellets. Consequently, the catalyst pellets are likely to undergo shrinkage or pulverization during the dehydrogenation reaction, whereby various troubles are likely to be caused by the change of the catalyst size or by the deterioration of the catalyst strength.

On the other hand, the catalysts prepared by calcining the iron oxide at a high temperature, are likely to lead to the formation of by-products such as toluene or benzene, and thus have a low selectivity for styrene, and the lower limit of the stream to oil ratio is limited to a level of about 6.

Japanese Unexamined Patent Publication No. 90102/1979 discloses that a catalyst containing an alkali metal oxide and vanadium oxide as promoters on spinel of $MgFe_{1.9}Cr_{0.1}O_4$ or $MnFe_{1.5}Cr_{0.5}O_4$ can be used at a steam to oil ratio of from 6 to 12. However, it is difficult or cumbersome to prepare such a catalyst, and it requires a high temperature for calcination. Therefore, it is difficult to use such a catalyst as an industrial catalyst.

As an industrially employed catalyst other than the above-mentioned iron-chromium-potassium type, there is an iron-cerium-molybdenum-potassium catalyst as disclosed in Japanese Unexamined Patent Publication No. 120887/1974 or No. 120888/1974. This catalyst has a feature that the styrene selectivity is high. However, it requires a potassium carbonate content as high as at least 40 wt.% in order to use it at a low steam to oil ratio, whereby it is impossible to avoid the above-mentioned drawbacks such that due to the hygroscopic nature of potassium or due to the migration of potassium during the dehydrogenation reaction, the catalyst tends to undergo the structural disintegration.

2

EP 0 177 832 B1

Thus, there is no industrial catalyst presently available that provides adequate stability in the catalytic activity and adequate yield of styrene at a low steam to oil ratio of 12 or less.

The present inventors have found it possible to obtain a catalyst which provides stability in the catalytic activity and high yield of styrene even under a dehydrogenation reaction condition with a low steam to oil ratio at a level of from 3.5 to 12, by incorporating magnesium into e.g. an iron-chromium-potassium catalyst or an iron-cerium-molybdenum-potassium catalyst. The present invention has been accomplished on the basis of this discovery.

The present invention provides a dehydrogenation catalyst consisting essentially of 15.0—35.6 wt.% of potassium oxide, 1.8—5.4 wt.% of magnesium oxide, a promoter selected from a) to e) stated below, and iron oxide for the rest:

a) $Cr_2O_3$ 1—4 wt.%;
b) $Cr_2O_3$ 1—4 wt.%, and $CaO$ 3.1—7.3 wt.%;
c) $Cr_2O_3$ 1—4 wt.%, $Ce_2O_3$ 3—6 wt.%, and $MoO_3$ 0.5—3 wt.%;
d) $Ce_2O_3$ 3—6 wt.%, and $MoO_3$ 0.5—3 wt.%; and
e) $Ce_2O_3$ 3—6 wt.%, $MoO_3$ 0.5—3 wt.%, and $CaO$ 3.1—7.3 wt.%.

The promoter can be selected from a) and b) stated below:

a) $Cr_2O_3$ 1.5—3.8 wt.% and $CaO$ 3.1—6.0 wt.%, and
b) $Cr_2O_3$ 1.5—3.8 wt.%, and $Ce_2O_3$ 3—6 wt.%, and $MoO_3$ 0.5—3.0 wt.%.

or from a) and b) stated below:

a) $Ce_2O_3$ 3—6 wt.%, and $MoO_3$ 0.5—3.0 wt.%, and
b) $Ce_2O_3$ 3—6 wt.%, and $MoO_3$ 0.5—3.0 wt.%, and $CaO$ 3.1—6.0 wt.%.

According to the present invention, the molded catalyst pellets are structurally stabilized by the addition of magnesium, and it is thereby possible to prevent the deterioration in the strength of the catalyst due to the absorption of water by potassium in the catalyst and simultaneously to prevent the disintegration of the catalyst due to the migration of potassium during the dehydrogenation reaction. Further, by limiting the amount of the magnesium to a level of from 1.8 to 5.4 wt.% as the oxide, it is possible to prevent the deterioration in the strength of the catalyst after the dehydrogenation reaction. Furthermore, it is now possible to control the amount of potassium oxide in the catalyst to a level of from 15.0 to 35.6 wt.%, which is substantially the same as the content in the conventional industrial catalysts which used to be employed at a steam to oil ratio of more than 12.

Heretofore, it has been known that even a catalyst having a potassium oxide content of about 35 wt.% can be stabilized in its pellet structure by calcination at a high temperature of from 700 to 900°C, whereby it tends to hardly undergo shrinkage or pulverization during the dehydrogenation reaction. However, it is hardly possible to avoid sintering of the catalyst by calcination, or the decrease of the catalytic activity due to a substantial decrease of the surface area.

Whereas, the catalyst containing magnesium oxide of the present invention is structurally stabilized, and even when the catalyst with a potassium oxide content of about 35 wt.% is subjected to calcination at a high temperature of at least 700°C, the pellet structure of the catalyst is further stabilized without deterioration of the catalytic activity. Namely, a magnesium-containing iron-chromium-potassium catalyst having the following composition exhibits a stabilized performance at a steam to oil ratio of from 3.5 to 12, whereby the conversion of ethylbenzene is higher than that attained by an iron-chromium-potassium catalyst containing 40.5 wt.% of potassium oxide, and the selectivity for styrene is substantially equal or higher.

| | |
|---|---|
| $Fe_2O_3$ | 55.0—82.2 wt.% |
| $Cr_2O_3$ | 1—4 wt.% |
| $K_2O$ | 12.0—35.6 wt.% |
| $MgO$ | 1.8—5.4 wt.% |

(The content of each component in the composition is represented by the weight % of its oxide. The same applies in the following description.)

An iron-chromium-potassium catalyst usually undergoes a substantial reduction in its surfaced area before and after the dehydrogenation reaction. (For instance, a catalyst containing 40.5 wt.% of potassium oxide undergoes a reduction in its surface area of about 30.5% and an increase in its average pore diameter of about 10%.) In particular, a catalyst containing at least 22.6 wt.% of potassium oxide undergoes a shrinkage of the pellet size by from 5 to 8% during the dehydrogenation reaction, or complete disintegration and pulverization (disintegration rate: 100%).

Whereas, the catalyst containing magnesium oxide of the present invention rather tends to have an increased surface area after the reaction, and the change of the average pore diameter of the catalyst is

3

small. Further, in the present invention, even when the catalyst contains at least 22.6 wt.% of potassium oxide, the shrinkage of the catalyst pellets after the reaction is as low as at most 4%, and the disintegration rate of the catalyst in boiling water is as low as at most 35%.

Thus, magnesium oxide has a function to stabilize the structure of the catalyst pellets. However, if the amount of magnesium oxide exceeds 5.4 wt.% the pore volume of the catalyst tends to be so large that the pulverization of the catalyst (attrition loss) of a level of from 20 to 40% takes place when subjected to the abrasion test after the dehydrogenation reaction. This attrition loss causes the pulverization of the catalyst when a substantial force is imparted to the catalyst during the reaction (for instance, when a part of the catalyst layer flows due to an excessive gas flow), such being undesirable. However, when the magnesium oxide content is limited within a range of from 1.8 to 5.4 wt.%, the attrition loss may be reduced to a level of about 15%, which is comparable with the conventional industrial catalyst, the effect of magnesium oxide for the stabilization of the catalyst pellet structure is not lowered, and the catalytic activity will be maintained. On the other hand, if the content of magnesium oxide is less than 1.8 wt.%, no adequate effect for the stabilization of the structure by magnesium oxide will be obtained.

From the observation by X-ray diffraction, it has been confirmed that a part of magnesium oxide in the catalyst forms a solid solution in iron oxide in the form of $MgFe_2O_4$, whereby spinel of $MgFe_{1.9}Cr_{0.1}O_4$ was not found. A magnesium-containing iron-chromium-potassium-calcium catalyst having the following composition has a feature that the selectivity for styrene is very high, although the conversion of ethylbenzene at a stream to oil ratio of from 3.5 to 12 is substantially the same as in the case of the iron-chromium-potassium-magnesium catalyst of the present invention.

$$
\begin{array}{ll}
Fe_2O_3 & 47.7\text{—}79.1 \text{ wt.\%} \\
Cr_2O_3 & 1\text{—}4 \text{ wt.\%} \\
K_2O & 15.0\text{—}35.6 \text{ wt.\%} \\
MgO & 1.8\text{—}5.4 \text{ wt.\%} \\
CaO & 3.1\text{—}7.3 \text{ wt.\%}
\end{array}
$$

The changes in the surface area and pore diameter of this catalyst before and after the dehydrogenation reaction are small, and the catalyst is structurally stable.

On the other hand, it is known that a iron-cerium-molybdenum-potassium catalyst has a high selectivity for styrene. When magnesium is added thereto to obtain a catalyst having the following composition, the catalyst exhibits stabilized performance at a stream to oil ratio of from 6 to 12, and the conversion of ethylbenzene is higher than that attainable by the iron-cerium-molybdenum-potassium catalyst containing 26.8 wt.% of potassium oxide, and the selectivity was equal or higher.

$$
\begin{array}{ll}
Fe_2O_3 & 50\text{—}79.7 \text{ wt.\%} \\
Ce_2O_3 & 3\text{—}6 \text{ wt.\%} \\
MoO_3 & 0.5\text{—}3 \text{ wt.\%} \\
K_2O & 15.0\text{—}35.6 \text{ wt.\%} \\
MgO & 1.8\text{—}5.4 \text{ wt.\%}
\end{array}
$$

The changes in the surface area and pore diameter of this catalyst before and after the reaction as well as the shrinkage of the catalyst pellets are small. No disintegration of the catalyst pellets in boiling water was observed. The attrition loss was small as compared with the catalyst containing more than 5.4 wt.% of magnesium oxide.

A magnesium-containing iron-cerium-molybdenum-potassium-calcium catalyst having the following composition exhibits stable performance at a steam to oil ratio of from 6 to 12, and the conversion of ethyl-benzene was equal or higher than that attained by the iron-cerium-molybdenum-potassium catalyst containing 26.8 wt.% of potassium oxide, and the selectivity is high.

$$
\begin{array}{ll}
Fe_2O_3 & 42.7\text{—}76.6 \text{ wt.\%} \\
Ce_2O_3 & 3\text{—}6 \text{ wt.\%} \\
MoO_3 & 0.5\text{—}3 \text{ wt.\%} \\
K_2O & 15.0\text{—}35.6 \text{ wt.\%} \\
MgO & 1.8\text{—}5.4 \text{ wt.\%} \\
CaO & 3.1\text{—}7.3 \text{ wt.\%}
\end{array}
$$

Further, a magnesium-containing iron-cerium molybdenum-potassium-chromium catalyst having the following composition exhibits stable high performance at a steam to oil ratio of from 3.5 to 12 and high selectivity. It has a feature that it shows particularly high selectivity at a steam to oil ratio of 6 or less and high activity at a steam to oil ratio of 6 or higher.

$$
\begin{aligned}
&Fe_2O_3 && 46\text{—}78.7 \text{ wt.}\% \\
&Ce_2O_3 && 3\text{—}6 \text{ wt.}\% \\
&MoO_3 && 0.5\text{—}3 \text{ wt.}\% \\
&K_2O && 15.0\text{—}35.6 \text{ wt.}\% \\
&Cr_2O_3 && 1\text{—}4 \text{ wt.}\% \\
&MgO && 1.8\text{—}5.4 \text{ wt.}\%
\end{aligned}
$$

Thus, it is evident that the dehydrogenation catalysts of the present invention have excellent catalytic activity, selectivity and structural stability.

Further, according to the present invention, even when the catalyst has a high content of potassium oxide, the structural stability of the catalyst pellets can be improved by calcination at a high temperature of at least 700°C, without impairing the catalytic activity.

Now, the present invention will be described in further detail with reference to Examples. The test for the catalytic activity was conducted by the following method unless otherwise specified.

Into a stainless steel reaction tube having an inner diameter of 32 mm, 50 cm³ of extrusion-molded catalyst was packed, and the reaction tube was heated by an electric furnace to conduct the reaction at a predetermined temperature.

As the reaction conditions, the pressure was atmospheric pressure, the liquid hourly space velocity of ethylbenzene was 1, and the steam to oil ratio was 6 or 10.

The conversion of ethylbenzene and the selectivity were calculated by the following formulas.

$$
\text{Conversion (\%) of ethylbenzene} = \frac{\begin{array}{l}\text{Ethylbenzene concentration at the inlet of the catalytic bed (wt \%)} - \text{Ethylbenzene concentration at the outlet of the catalytic bed (wt.\%)}\end{array}}{\text{Ethylbenzene concentration at the inlet of the catalytic bed (wt.\%)}} \times 100
$$

$$
\text{Selectivity for styrene (\%)} = \frac{\text{Amount of formed styrene}}{\text{Amount of reacted ethylbenzene}} \times 100
$$

Example 1

500 g of α-ferric oxide, 26 g of chromic oxide, 307 g of potassium carbonate and 44 g of magnesium carbonate (commercially available basic magnesium carbonate) were mixed. After an addition of pure water, the mixture was thoroughly mixed by a kneader to obtain an extrudable paste. This paste was extrusion-molded with a diameter of 3,17 mm (⅛ inch), then dried and calcined at 540°C for 6 hours to obtain a catalyst having the following composition.

$$
\begin{aligned}
&Fe_2O_3 && 66.4 \text{ wt.}\% \\
&Cr_2O_3 && 3.5 \text{ wt.}\% \\
&K_2O && 27.7 \text{ wt.}\% \\
&MgO && 2.4 \text{ wt.}\%
\end{aligned}
$$

The dehydrogenation test data are presented in Tables 1 and 2.

In Table 1, "Temp-50%" means a temperature of the catalyst layer which gives a conversion of ethylbenzene of 50%, and "Selec-50%" means a selectivity for styrene at a conversion of ethylbenzene of 50%.

Referring to Table 2, the disintegration rate of pellets in boiling water was measured in such a manner that about 10 g of the catalyst was placed in 50 ml of boiling water, followed by evaporation to dryness, and it was calculated in accordance with the following formula.

$$
\text{Disintegration rate of pellets in boiling water (\%)} = \frac{\text{Weight of disintegrated catalyst}}{\text{Weight of total catalyst dried}} \times 100
$$

# EP 0 177 832 B1

The changes of physical properties such as the surface area, pore volume, pore diameter and pellet diameter before and after the reaction was obtained in accordance with the following formula.

$$\text{The change of physical properties before and after reaction (\%)} = \frac{\text{Physical properties before reaction} - \text{Physical properties after reaction}}{\text{Physical properties before reaction}} \times 100$$

The attrition loss after the reaction was obtained in such a manner that about 10 g of the catalyst after the reaction was placed in a horizontal cylinder and rotated for abrasion test, and the pulverized catalyst was sieved with a 2 mm (10 mesh) sieve. The amount of the remained catalyst was measured, and the attrition loss was obtained in accordance with the following formula.

$$\text{Attrition loss (\%)} = \frac{\text{Weight before sieving} - \text{Weight after sieving}}{\text{Weight before sieving}} \times 100$$

## Example 2

A catalyst was prepared in the same manner as in Example 1 except that 29 g of chromic oxide, 385 g of potassium carbonate and 48 g of magnesium carbonate were used.

$$
\begin{array}{ll}
Fe_2O_3 & 61.6 \text{ wt.\%} \\
Cr_2O_3 & 3.6 \text{ wt.\%} \\
K_2O & 32.3 \text{ wt.\%} \\
MgO & 2.5 \text{ wt.\%}
\end{array}
$$

The dehydrogenation test data are presented in Tables 1 and 2.

## Example 3

A catalyst was prepared in the same manner as in Example 2 except that the calcination was conducted at 750°C for 2 hours.

The dehydrogenation test data are presented in Tables 1 and 2.

## Example 4

A catalyst was prepared in the same manner as in Example 1 except that 28 g of chromic oxide, 278 g of potassium carbonate, 46 g of magnesium carbonate and 74 g of calcium carbonate were used.

$$
\begin{array}{ll}
Fe_2O_3 & 64.3 \text{ wt.\%} \\
Cr_2O_3 & 3.6 \text{ wt.\%} \\
K_2O & 24.3 \text{ wt.\%} \\
MgO & 2.5 \text{ wt.\%} \\
CaO & 5.3 \text{ wt.\%}
\end{array}
$$

.The dehydrogenation test data are presented in Tables 1 and 2.

## Comparative Example 1

A catalyst was prepared in the same manner as in Example 1 except that 500 g of α-ferric oxide, 32 g of chromic oxide and 532 g of potassium carbonate was used.

$$
\begin{array}{ll}
Fe_2O_3 & 55.9 \text{ wt.\%} \\
Cr_2O_3 & 3.6 \text{ wt.\%} \\
K_2O & 40.5 \text{ wt.\%}
\end{array}
$$

The dehydrogenation test data are presented in Tables 1 and 2.

## Comparative Example 2

A catalyst was prepared in the same manner as in Example 1 except that 32 g of chromic oxide, 320 g of potassium carbonate and 212 g of megnesium carbonate were used.

$$
\begin{array}{ll}
Fe_2O_3 & 59.6 \text{ wt.\%} \\
Cr_2O_3 & 3.8 \text{ wt.\%} \\
K_2O & 25.9 \text{ wt.\%} \\
MgO & 10.7 \text{ wt.\%}
\end{array}
$$

The dehydrogenation test data are shown in Tables 1 and 2.

6

## Example 5

A catalyst was prepared in the same manner as in Example 1 except that 500 g of α-ferric oxide, 43 g of cerium oxide, 22 g of molybdenum oxide, 261 g of potassium carbonate and 43 g of magnesium carbonate were used.

$Fe_2O_3$  65.7 wt.%
$Ce_2O_3$  5.7 wt.%
$MoO_3$  2.9 wt.%
$K_2O$  23.3 wt.%
$MgO$  2.4 wt.%

The dehydrogenation test data are presented in Tables 1 and 2.

## Example 6

A catalyst was prepared in the same manner as in Example 1 except that 500 g of α-ferric oxide, 51 g of cerium oxide, 25 g of molybdenum oxide, 303 g of potassium carbonate and 51 g of magnesium carbonate and 81 g of calcium carbonate were used.

$Fe_2O_3$  58.9 wt.%
$Ce_2O_3$  6.0 wt.%
$MoO_3$  3.0 wt.%
$K_2O$  24.3 wt.%
$MgO$  2.5 wt.%
$CaO$  5.3 wt.%

The dehydrogenation test data are shown in Tables 1 and 2.

## Example 7

A catalyst was prepared in the same manner as in Example 1 except that 500 g of α-ferric oxide, 46 g of cerium oxide, 23 g of molybdenum oxide, 275 g of potassium carbonate and 46 g of magnesium carbonate and 28 g of chromic oxide were used.

$Fe_2O_3$  62.3 wt.%
$Ce_2O_3$  5.7 wt.%
$MoO_3$  2.9 wt.%
$K_2O$  23.3 wt.%
$Cr_2O_3$  3.4 wt.%
$MgO$  2.4 wt.%

The dehydrogenation test data are shown in Tables 1 and 2.

## Comparative Example 3

A catalyst was prepared in the same manner as in Example 1 except that 500 g of α-ferric oxide, 42 g of cerium oxide, 21 g of molybdenum oxide and 277 g of potassium carbonate were used.

$Fe_2O_3$  66.5 wt.%
$Ce_2O_3$  5.6 wt.%
$MoO_3$  2.8 wt.%
$K_2O$  25.1 wt.%

The dehydrogenation test data are presented in Tables 1 and 2.

## Comparative Example 4

A catalyst was prepared in the same manner as in Example 1 except that 500 g of α-ferric oxide, 59 g of cerium oxide, 29 g of molybdenum oxide, 353 g of potassium carbonate and 235 g of magnesium carbonate were used.

$Fe_2O_3$  53.9 wt.%
$Ce_2O_3$  6.3 wt.%
$MoO_3$  3.2 wt.%
$K_2O$  25.9 wt.%
$MgO$  10.7 wt.%

The dehydrogenation test data are shown in Tables 1 and 2.

**EP 0 177 832 B1**

Comparative Example 5

A catalyst was prepared in the same manner as in Example 1 except that 500 g of α-ferric oxide, 42 g of cerium oxide, 21 g of molybdenum oxide, 252 g of potassium carbonate and 25 g of magnesium carbonate were used.

$Fe_2O_3$    67.1 wt.%
$Ce_2O_3$    5.6 wt.%
$MoO_3$    2.8 wt.%
$K_2O$    23.0 wt.%
$MgO$    1.4 wt.%

The dehydrogenation test data are shown in Tables 1 and 2.

Table 1

| Catalyst | Steam to oil ratio (molar ratio) | Temp-50% (°C)* | Selec-50% (%) ** |
|---|---|---|---|
| Example 1 | 6 | 580 | 93.1 |
| Example 2 | 6 | 582 | 93.7 |
| Example 3 | 6 | 586 | 94.1 |
| Example 4 | 6 | 596 | 94.6 |
| Comparative Example 1 | 6 | 603 | 93.4 |
| Comparative Example 2 | 6 | 593 | 93.9 |
| Example 5 | 10 | 584 | 97.1 |
| Example 6 | 10 | 589 | 98.0 |
| Example 7 | 10 | 578 | 96.7 |
| Comparative Example 3 | 10 | 592 | 97.4 |
| Comparative Example 4 | 10 | 580 | 96.7 |
| Comparative Example 5 | 10 | 590 | 97.0 |

\* Temp-50%: Temperature of the catalyst layer which gives a conversion of ethylbenzene of 50%.

\*\* Selec-50%: Selectivity for styrene at a conversion of ethylbenzene of 50%.

8

Table 2

| Catalyst | Disintegration rate of pellets in boiling water (%) | Change rates of physical properties before and after the reaction (%) | | | | Attrition loss after the reaction (%) |
|---|---|---|---|---|---|---|
| | | Surface area | Pore volume | Pore diameter | Pellet diameter | |
| Example 1 | 79 | −88 | 0 | 0 | 2.0 | 8.1 |
| Example 2 | 80 | −13 | +13 | +15 | 3.4 | 14.6 |
| Example 3 | 35 | −30 | +15 | −2 | 2.9 | 13.8 |
| Example 4 | 0 | −32 | +7 | −4 | 2.2 | 8.9 |
| Comparative Example 1 | 100 | +29 | +21 | −10 | 6.2 | 12.7 |
| Comparative Example 2 | 0 | −20 | −20 | +5 | 2.2 | 21.1 |
| Example 5 | 0 | −9 | +9 | −10 | 1.1 | 8.7 |
| Example 6 | 0 | −22 | −8 | −3 | 0.7 | 10.3 |
| Example 7 | 0 | +10 | 0 | −13 | 1.5 | 9.8 |
| Comparative Example 3 | 100 | +53 | +12 | −120 | 6.7 | 15.3 |
| Comparative Example 4 | 0 | +6 | +4 | −2 | 1.2 | 24.0 |
| Comparative Example 5 | 36 | +21 | +25 | −18 | 3.0 | 16.4 |

EP 0 177 832 B1

**Claims**

1. A dehydrogenation catalyst consisting of 15.0—35.6 wt.% of potassium oxide, 1.8—5.4 wt.% of magnesium oxide, a promoter selected from a) to e) stated below, and iron oxide for the rest:

a) $Cr_2O_3$ 1—4 wt.%;
b) $Cr_2O_3$ 1—4 wt.%, and CaO 3.1—7.3 wt.%;
c) $Cr_2O_3$ 1—4 wt.%, $Ce_2O_3$ 3—6 wt.%, and $MoO_3$ 0.5—3 wt.%;
d) $Ce_2O_3$ 3—6 wt.%, and $MoO_3$ 0.5—3 wt.%; and
e) $Ce_2O_3$ 3—6 wt.%, $MoO_3$ 0.5—3 wt.%, and CaO 3.1—7.3 wt.%.

2. The catalyst in accordance with Claim 1, wherein the promoter is selected from a) and b) stated below:

a) $Cr_2O_3$ 1.5—3.8 wt.% and CaO 3.1—6.0 wt.%, and
b) $Cr_2O_3$ 1.5—3.8 wt.%, and $Ce_2O_3$ 3—6 wt.%, and $MoO_3$ 0.5—3.0 wt.%.

3. A composition in accordance with Claim 1, wherein the promoter is selected from a and b) stated below:

a) $Ce_2O_3$ 3—6 wt.%, and $MoO_3$ 0.5—3.0 wt.%, and
b) $Ce_2O_3$ 3—6 wt.%, and $MoO_3$ 0.5—3.0 wt.%, and CaO 3.1—6.0 wt.%.

**Patentansprüche**

1. Dehydrierungskatalysator, bestehend aus 15,0 bis 35,6 Gew.-% Kaliumoxid, 1,8 bis 5,4 Gew.-% Magnesiumoxid, einem Promotor, ausgewählt aus a) bis e) wie nachstehend angegeben, und Eisenoxid (Rest):

a) $Cr_2O_3$ 1—4 Gew.-%;
b) $Cr_2O_3$ 1—4 Gew.-%, und CaO 3,1—7,3 Gew.-%;
c) $Cr_2O_3$ 1—4 Gew.-%, $Ce_2O_3$ 3—6 Gew.-%; und $MoO_3$ 0,5—3 Gew.%;
d) $Ce_2O_3$ 3—6 Gew.-%; und $MoO_3$ 0,5—3 Gew.-%; und
e) $Ce_2O_3$ 3—6 Gew.-%; und $MoO_3$ 0,5—3 Gew.-%, und CaO 3,1—7,3 Gew.-%;

2. Katalysator nach Anspruch 1, worin der Promotor aus a) und b) wie nachstehend angegeben, ausgewählt ist:

a) $Cr_2O_3$ 1,5—3,8 Gew.-%, und CaO 3,1—6,0 Gew.-% und
b) $Cr_2O_3$ 1,5—3,8 Gew.-%, und $Ce_2O_3$ 3—6 Gew.-% und $MoO_3$ 0,5—3,0 Gew.-%.

3. Zusammensetzung nach Anspruch 1, worin der Promotor aus a) und b), wie nachstehend angegeben, ausgewählt ist:

a) $Ce_2O_3$ 3—6 Gew.-%; und $MoO_3$ 0,5—3,0 Gew.-%; und
b) $Ce_2O_3$ 3—6 Gew.-%; und $MoO_3$ 0,5—3,0 Gew.-%; und CaO 3,1—6,0 Gew.-%.

**Revendications**

1. Catalyseur de déshydrogénation consistant en 15,0 à 35,6 parties pour cent en poids d'oxyde de potassium, 1,8 à 5,4 parties pour cent en poids d'oxyde de magnésium, un agent promoteur choisi parmi a) à e) énumérés ci-après, le reste étant de l'oxyde de fer:

a) $Cr_2O_3$ 1—4% en poids
b) $Cr_2O_3$ 1—4% en poids et CaO 3,1—7,3% en poids
c) $Cr_2O_3$ 1—4% en poids, $Ce_2O_3$ 3—6% en poids, et $MoO_3$ 0,5—3% en poids
d) $Ce_2O_3$ 3—6% en poids, et $MoO_3$ 0,5—3% en poids et
e) $Ce_2O_3$ 3—6% en poids, $MoO_3$ 0,5—3% en poids et CaO 3,1—7% en poids.

2. Catalyseur suivant la revendication 1, dans lequel l'agent promoteur est choisi parmi a) et b) ci-dessous:

a) $Cr_2O_3$ 1,5—3,8% en poids, et CaO 3,1—6,0% en poids et
b) $Cr_2O_3$ 1,5—3,8% en poids, et $Ce_2O_3$ 3—6% en poids, et $MoO_3$ 0,5—3,0% en poids.

3. Composition suivant la revendication 1, dans laquelle l'agent promoteur est choisi parmi a) et b) ci-dessous:

a) $Ce_2O_3$ 3—6% en poids, et $MoO_3$ 0,5—3,0% en poids, et
b) $Ce_2O_3$ 3—6% en poids, et $MoO_3$ 0,5—3,0% en poids et CaO 3,1—6,0% en poids.

11